# EUROPEAN PATENT APPLICATION

(11) **EP 1 293 194 A1**
(43) Date of publication of application: **19.03.2003**
(21) Application number: 02256442.1
(22) Date of filing: 17.09.2002
(51) Int. Cl.: A61K 9/00

(54) **Physically stable sprayable gel composition**

(30) Priority: 18.09.2001 US 954911
(71) Applicant: Johnson & Johnson Consumer Companies, Inc., Skillman, NJ 08558-9418 (US)
(72) Inventor: Kulkarni, Arun B., East Burnswick, NJ 08816 (US); Pascal, Felipe A., Hillsborough, NJ 08844-4361 (US); Halas, Lynn Ann, Ewing, NJ 08638 (US)
(74) Representative: Mercer, Christopher Paul

(57) **Abstract**

A physically stable sprayable gel composition useful for delivering actives to skin including: a gelling agent; water; at least one water-miscible solvent; and a viscosity stabilizer is disclosed.

## Description

### Background of the Invention

### Field of the Invention

The present invention relates to a physically stable sprayable gel composition. The composition includes a gelling agent, water, a water-miscible solvent, and a viscosity stabilizer. Active ingredients, such as anti-itch actives, topical anesthetics, anti-inflammatory agents, antipruritics, and cooling actives may be included in the compositions.

### Description of the Prior Art

Sunburn, bug bites, and poison ivy are conditions that cause discomfort for many consumers. Consumers purchase many products for relieving the itch, irritation, and inflammation associated with these conditions. Some of the products also contain cooling agents to help relieve the discomfort. The products typically contain actives such as menthol, camphor, diphenhydramine, calamine and the like.

The anti-itch products that are commercially available tend to be in the form of liquids or lotions that are sprayed onto or rubbed into the affected area of the skin. Liquids which are intended to be sprayed tend to have a viscosity similar to that of water. This means that sprayable liquids tend to spread too easily, can drip off the sprayed skin, and can be messy with respect to articles of clothing.

Commercially available anti-itch lotions tend to be much more viscous than liquid sprays. The lotions may be difficult to pour and to spread on the skin. Frequently, the lotions have an undesirable color, due to the color of one of the ingredients therein. This can be unsightly, as the area of skin where the lotion is applied takes on the undesirable color. Color is also problematic for staining articles of clothing.

Gel compositions have been developed for treating sunburn and the itch associated with poison ivy. These gel compositions utilize clays to form the gel. The gels tend to be highly viscous. The high viscosity is desirable, as it prevents the composition from running or dripping. The gels are typically sold in squeeze tubes. We have found that for various reasons, consumers prefer a sprayable composition for the treatment of sunburn, bug bites, poison ivy and the like.

Shear thinning gels are also known and taught for example in clay product formularies. These gels have a high inherent viscosity. The viscosity is reduced under shear, such as when pumped through a spray pump. This is known as shear thinning. The shear thinning allows the gel to be sprayed due to the reduced viscosity. After being sprayed, the gel returns to its original high viscosity. To our knowledge, sprayable gels are commercially available for use in baby products, hair care products, and after sun products.

One problem associated with known sprayable gel compositions is that the compositions are not physically stable. By physically stable is meant that the composition does not phase separate, increase in viscosity unacceptably, or decrease in viscosity unacceptably over a period of use typically associated with commercial products. Compositions that are not physically stable are problematic for several reasons. The composition might become too viscous to spray. The composition could also become too thin and tend to run or drip. Phase separation can also occur, leading to inappropriate concentrations of active ingredient being delivered. The stability of sprayable gel compositions may be adversely affected by the incorporation of active ingredients into the compositions.

Therefore, there is a need for a physically stable sprayable gel composition that is useful for delivering actives to skin, such as anti-itch actives and cooling actives.

### Summary of the Invention

We have discovered that it is possible to provide a physically stable sprayable gel composition for use in delivering actives to skin. The present invention provides a physically stable sprayable gel composition useful for delivering actives to skin which includes a gelling agent; water; at least one water-miscible solvent; and at least one viscosity stabilizer.

### Detailed Description of Preferred Embodiments

The compositions of the present invention include a gelling agent. Suitable gelling agents include, but are not limited to, clays. The clays may be isolated from nature or may be synthesized based on naturally occurring clay compositions. The amount of gelling agent utilized in the compositions of the present invention may range from about 0.4 percent to about 2 percent, preferably from about 0.7 percent to about 1.5 percent by weight, based on the total weight of the composition.

The term "smectite" refers to a class of clays that swell in water. Suitable smectite clays useful in this invention include, but are not limited to, aluminosilicates such as bentonite, montmorillonite, hectorite, sucinite, saponite, nontronite, vermiculite, beidellite, and stevensite. Synthetic montmorillonite clay commercially available, e.g., under the Tradename Laponite, is preferred for use in the practice of the present invention.

The structure of the clays useful in the invention may be of the pyrophillite (dioctahedral) type or the talc (trioctahedral) type. Mixed layer clays, where at least one layer is a smectite, are also useful in this invention. Other clays which may be used in the compositions of the present invention include, but are not limited to, sepiolite, palygorskite, and zeolites. Amorphous clays may also be useful in the present invention. The term amorphous clay means clay that does not have an ordered structure. This group of clays includes, but is not limited to, allophane and imogolite.

The compositions of the present invention also contain water. The source of water is not critical. Suitable sources include tap water, deionized water, and the like. The viscosity of the compositions of the invention may, however, be affected by ions present in the water; therefore deionized water is preferred. The amount of water in compositions of the present invention ranges from about 25 percent to about 65 percent by weight, preferably from about 40 percent to about 60 percent by weight, based on the total weight of the composition.

At least one water-miscible solvent is included in the compositions of the present invention. Suitable water-miscible solvents include, but are not limited to, alcohols, such as ethanol, isopropanol and the like; glycols, such as propylene glycol, dipropylene glycol, glycerol, and the like; and esters such as Triacetin, and the like; and combinations thereof. Ethanol, glycerol, and combinations thereof are preferred as water-miscible solvents for use in the practice of the present invention. The amount of water-miscible solvent may range from about 30 percent to about 65 percent by weight, preferably from about 35 percent to about 50 percent by weight, based on the total weight of the composition.

At least one viscosity stabilizer is utilized in the compositions of the present invention to provide a physically stable gel. Suitable viscosity stabilizers include, but are not limited to, salts, such as sodium chloride, potassium chloride, calcium chloride, sodium citrate, and the like; acids, such as citric acid, formic acid, malic acid, maleic acid, ethylenediamine tetracetic acid, and the like; sugars, such as glucose, sucrose, fructose, and the like; starches; and combinations thereof. The amount of viscosity stabilizer may range from about 0.001 percent to about 0.1 percent by weight, preferably from about 0.01 percent to about 0.05 percent by weight, based on the total weight of the composition.

The compositions of the present invention are suitable for delivering active ingredients to the skin. Active ingredients which may be incorporated into compositions of the present invention include antihistamines, anti-fungal agents, anti-inflammatory agents, antipruritics, Skin and mucous membrane treatment agents, wound care agents, anesthetics, topical analgesics, and natural active ingredients. Active ingredients include, but are not limited to, menthol, camphor, menthyl lactate, diphenhydramine; Centaurea Cyanus, Kalmia Latifolia, Mandragora Vernalis, Tanacetum Parthenium, Acacia Catechu, Aloe Barbadensis, Convallaria Majalis, Echinacea, Eucalyptus, Mentha Piperita, Rosa Canina, Sassafras Albidum, Fragaria Vesca, Matricaria Chamomilla, Salvia Officinalis, Anagallis Arvensis, Oenothera Biennis, Verbena Officinalis, Aesculus Hippocastanum, Avena Sativa, Baptista Tinctoria, Digitalis Purpurea, Hamamelis Virginiana, Helianthus Annuus, Hypericum Perforatum, Lawsonia Inermis, Nerium Odoratum, Calendula Officinalis, Cinnamomum Verum, Coffea Arabica, Cola Acuminata, Solidago Species, and combinations thereof. Menthol, camphor, menthyl lactate, and mixtures thereof are particularly preferred if active ingredients are to be included in the inventive composition. The amount of active agent in the composition of the present invention may range from about 0.1 percent by weight to about 3 percent, preferably from about 0.25 percent to about 2.0 percent by weight, based on the total weight of the composition.

Thickening agents may be utilized to adjust the viscosity of the compositions of the present invention. Suitable thickening agents include, but are not limited to, carboxymethyl cellulose, methyl cellulose, hydroxyethyl cellulose and shear sensitive polysaccharide gums, such as xanthan gum, guar gum, karaya gum and the like. The amount of thickening agent added may range from about 0.001 percent to about 0.1 percent by weight, preferably from about 0.01 percent to about 0.05 percent by weight, based on the total weight of the composition.

Emollients may also be included in the compositions of the present invention. Suitable emollients include, but are not limited to, glycerol, propylene glycol, hexylene glycol, butylene glycol, dipropylene glycol, and mixtures thereof. The amount of emollient added may range from about 1 percent to about 10 percent, preferably from about 2 percent to about 8 percent by weight, based on the total weight of the composition.

As is known in the art, preservatives such as benzyl alcohol, phenoxyethanol, and the like may be utilized in effective amounts.

The compositions of the present invention are prepared by admixing the ingredients in a suitable vessel and stirring the mixture until uniform. When shear is applied to the compositions of the present invention, such as when the compositions are pumped through a spray pump, the viscosity of the composition decreases. Upon settling, the inherent viscosity of the composition returns.

Several examples are described below to illustrate the present invention. The invention should not be construed as being limited to the details thereof.

Samples were prepared as set forth in Tables 1, 2, 3, 4, and 5 hereinbelow. Samples were stored at 40°C and checked for physical stability after 13 weeks of storage. Samples were considered stable if there was no phase separation and the sample could be pumped from the sprayer in which they had been aged. Samples were also tested for freeze/thaw stability. The samples were frozen and thawed for 3 cycles. The samples were considered stable if the sample, when returned to room temperature, had no phase separation and could be pumped from the sprayer.

**Table I**

| Sample 1 Ingredients | Function | % (w/w) |
|---|---|---|
| Water | - | 49.65 |
| Ethyl alcohol | Water miscible solvent | 42.75 |
| Glycerol | Emollient | 4.00 |
| Laponite XLG | Gelling agent | 1.00 |
| Menthyl Lactate | Active/Cooling agent | 1.00 |
| Menthol | Active/Topical analgesic | 0.50 |
| Camphor | Active/Topical analgesic | 0.50 |
| Benzyl Alcohol | Preservative | 0.50 |
| Fragrance | - | 0.05 |
| Citric Acid | Viscosity stabilizer | 0.03 |
| Xanthan Gum | Thickener | 0.02 |
| Total | - | 100.00 |

Sample 1 was a dispersion having the composition given in Table I. The dispersion was prepared according to the following procedure:

The dispersion of Sample 1 was prepared in a stainless steel container equipped with a stirrer. The Laponite XLG clay was added to the water and stirred under vigorous stirring for 20 minutes to form a dispersion of the clay in water. The glycerin and xantham gum were then added with stirring for 10 minutes. The ethyl alcohol, benzyl alcohol, camphor, menthol, menthyl lactate, fragrance and citric acid were combined in a separate container and mixed until a uniform solution was obtained. The aforementioned solution was slowly added to the aqueous clay dispersion with stirring. Stirring was continued for 10 minutes to yield the finished product. The finished product was transferred to pump spray containers. The dispersion was physically stable after 13 weeks at 40°C.

Samples 2 and 3, neither of which includes a viscosity stabilizer, are comparative examples in which the compositions were not physically stable. The compositions of samples 2 and 3 are given in Tables II and III, respectively. These compositions tended undesirably to produce a ringing gel upon aging. After aging for 4 weeks at 50°C, they could not be dispensed from a pump spray bottle.

**Table II**

| Sample 2 Ingredients | Function | % (w/w) |
|---|---|---|
| Water | - | 47.90 |
| Ethyl alcohol | Water miscible solvent | 43.61 |
| Glycerol | Emollient | 5.00 |
| Laponite XLG | Gelling agent | 2.00 |
| Menthol | Active/Topical analgesic | 1.00 |
| Camphor | Active/Topical analgesic | 0.50 |
| Total | - | 100.00 |
| Note: the preparation procedure followed that of Sample I | | |

**Table III**

| Sample 3 Ingredients | Function | % (w/w) |
|---|---|---|
| Water | - | 48.00 |
| Ethyl alcohol | Water miscible solvent | 42.75 |
| Glycerol | Emollient | 5.00 |
| Laponite XLG | Gelling agent | 1.50 |
| Menthyl Lactate | Active/Cooling agent | 1.00 |
| Menthol | Active/Topical analgesic | 1.00 |
| Camphor | Active/Topical analgesic | 0.50 |
| Fragrance | - | 0.05 |
| Xanthan Gum | Thickener | 0.20 |
| Total | - | 100.00 |
| Note: the preparation procedure followed that of Sample I | | |

The use of thickeners, such as xantham gum or cellulosics, such as carboxymethyl cellulose, help mitigate the formation of a rigid gel. However, if the concentration of the thickener is too high, the composition will not dispense through a pump spray bottle. Additionally, a high concentration of thickener has a negative effect on the clarity of the compositions.

The tendency of the composition to form a rigid gel can be mitigated by the addition of salts or acid to the compositions. However if the concentration of salt is too high, the clay particles will flocculate, creating clustered particles, which will tend to precipitate out under the influence of gravity. The amount of salt should be such that the clay network continues to occupy the whole container, preventing sedimentation. Sample 4 is an example of a suitable amount of an acid (i.e., citric acid) in a physically stable sprayable gel. The ingredients are listed in Table IV.

**Table IV**

| Sample 4 Ingredients | Function | % (w/w) |
|---|---|---|
| Water | | 49.17 |
| Ethyl alcohol | Water miscible solvent | 42.75 |
| Glycerol | Emollient | 4.00 |
| Laponite XLG | Gelling agent | 1.00 |
| Menthyl Lactate | Active/Cooling agent | 1.00 |
| Menthol | ActiveTopical analgesic | 1.00 |
| Camphor | Active/Topical analgesic | 0.50 |
| Benzyl Alcohol | Preservative | 0.50 |
| Fragrance | - | 0.05 |
| Citric Acid | Viscosity stabilizer | 0.03 |
| Total | | 100.00 |

pH of Sample 4 was 6.44

Sample 5 is an example of a composition in accordance with the present invention and having an anti-inflammatory agent incorporated therein. The composition of Sample 5 is listed in Table V.

**Table V**

| Sample 5 Ingredients | Function | % (w/w) |
|---|---|---|
| Water | | 50.20 |
| Ethyl alcohol | Water miscible solvent | 42.75 |
| Glycerol | Emollient | 4.00 |
| Laponite XLG | Gelling agent | 1.00 |
| Hydrocortisone | Active/Anti-inflammatory | 1.00 |
| Menthyl Lactate | Active/Cooling agent | 1.00 |
| Xanthan Gum | Thickener | 0.02 |
| Citric acid | Viscosity stabilizer | 0.03 |
| Total | | 100.00 |

The viscosity of Sample 1 was measured as a function of shear rate to demonstrate that the compositions of the present invention are shear thinning. A Brookfield LVT viscometer was utilized for the viscosity measurements. Spindle #2 was utilized and the viscosity was measured at 0.3, 0.6, 1.5, 3, 6, and 12 rotations per minute ("rpm"). The results are shown in Table VI.

**Table VI**

| rpm | Viscosity (cps) |
|---|---|
| 0.3 | 38,500 |
| 0.6 | 23,500 |
| 1.5 | 10,400 |
| 3 | 5,500 |
| 6 | 2,850 |
| 12 | 1,475 |

## Claims

1. A physically stable sprayable gel composition useful for delivering actives to skin comprising:
a gelling agent;
water;
at least one water-miscible solvent; and
at least one viscosity stabilizer selected from the group consisting of salts, acids, sugars, starches and combinations thereof.

2. The composition according to claim 1 wherein the gelling agent is a clay.

3. The composition according to claim 2 wherein the amount of clay ranges from 0.4 percent to 2 percent by weight, preferably from 0.7 percent to 1.5 percent by weight, based on the total weight of the composition.

4. The composition according to any one of claims 1 to 3 wherein the water-miscible solvent is selected from the group consisting of alcohols, glycols, esters, and combinations thereof.

5. The composition according to claim 4 wherein the water-miscible solvent is selected from ethanol, glycerol, and combinations thereof.

6. The composition according to claim 4 or claim 5 wherein the amount of water-miscible solvent ranges from 30 percent to 65 percent by weight, preferably from 35 percent to 50 percent by weight, based on the total weight of the composition.

7. The composition according to any one of claims 1 to 6 wherein the viscosity stabilizer is selected from citric acid, xanthan gum, and combinations thereof.

8. The composition according to claim 1 wherein the amount of viscosity stabilizer ranges from 0.001 percent to 0.1 percent by weight, preferably from 0.01 percent to 0.05 percent by weight, based on the total weight of the composition.

9. The composition according to any one of claims 1 to 8 further comprising an active ingredient seleced from the group consisitng of menthol, camphor, diphenhydramine, antifungal agents, antihistamine agents, anti-infective agents, anti-inflammatory agents, antipruritic agents, skin and mucous membrane agents, wound care agents, and combinations thereof.

10. The composition according to claim 9 wherein the active ingredient is selected from menthol, camphor, and combinations thereof.

11. The composition according to claim 9 or claim 10 wherein the amount of active ingredient ranges from 0.1 percent to 1 percent by weight, preferably from 0.25 percent to 0.75 percent by weight, based on the total weight of the composition.
